# EUROPEAN PATENT APPLICATION

(11) **EP 4 082 998 A1**
(43) Date of publication of application: **02.11.2022**
(21) Application number: 20904731.5
(22) Date of filing: 09.11.2020
(51) Int. Cl.: C07C 29/149, C07C 35/14, B01J 23/62

(54) **METHOD FOR PRODUCING 1,4-CYCLOHEXANEDIMETHANOL**

(30) Priority: 27.12.2019 KR 20190176139
(71) Applicant: Hanwha Solutions Corporation, Jung-gu Seoul 04541 (KR)
(72) Inventor: KIM, Eun Jeong, Daejeon 34128 (KR); LEE, Sun Uk, Daejeon 34128 (KR); LEE, Jong Kwon, Daejeon 34128 (KR); JANG, Namjin, Daejeon 34128 (KR)
(74) Representative: Berggren Oy
(86) International application number: PCT/KR2020/015572
(87) International publication number: WO 2021/132876

(57) **Abstract**

The present disclosure relates to a method for producing 1,4-cyclohexanedimethanol (CHDM) and, more specifically, provides a method for producing 1,4-cyclohexanedimethanol having a high proportion of trans isomers even without including an isomerization step, by adjusting the content of 1,4-cyclohexane dicarboxylic acid (CHDA) which is a reactant.

## Description

### [TECHNICAL FIELD]

### CROSS-REFERENCE TO RELATED APPLICATION(S)

This application claims the benefit of Korean Patent Application No. 10-2019-0176139 filed on December 27, 2019 with the Korean Intellectual Property Office, the content of which is incorporated herein by reference in its entirety.

The present disclosure relates to a method for producing 1,4-cyclohexanedimethanol (CHDM).

### [BACKGROUND ART]

1,4-Cyclohexanedimethanol (CHDM) is widely used as a raw material for medicines, synthetic resins, synthetic fibers, or dyes, and particularly, is used as a raw material for polyethylene terephthalate, which is an environmentally friendly polyester.

1 ,4-Cyclohexanedimethanol exists as stereoisomers of cis- and trans-forms, and in order to realize higher quality product, it is required to have higher ratio of trans 1,4-cyclohexanedimethanol (trans CHDM) than cis CHDM.

In a conventional method for producing CHDM, an isomerization reaction is usually involved using a method of performing an isomerization reaction of 1,4-cyclohexane dicarboxylic acid (CHDA) to increase the trans content, followed by a hydrogenation reaction with CHDM, a method of subjecting CHDA to a hydrogenation reaction to produce CHDM and then performing an isomerization reaction to produce CHDM with increased trans content, and the like. However, the conventional method of additionally performing the isomerization reaction has disadvantages in that the process is complicated and inefficient, and additional production costs are required, which are not commercially desirable.

Therefore, research on the CHDM production method capable of obtaining a high ratio of trans CHDM without progressing the isomerization reaction is underway. As an example, in International Patent Publication No. 2019-046412, an attempt was made to increase the content of trans CHDM that is produced by adjusting the ratio of tin and ruthenium of the hydrogenation catalyst used in the reaction of CHDA, but in a fixed bed reactor including a catalyst, crystallization proceeds easily in the conversion of CHDA which is a reactant, wherein there is a problem that the performance of the catalyst is reduced by the crystal, and the desired yield and the ratio of trans CHDM cannot be achieved. In addition, in Korean Patent No. 10-1639487, a simplified reactor was invented to minimize by-products, but apart from the fact that the purity of the product is improved, the proportion of trans CHDM in the product is unsatisfactory.

### [Prior Art Literature]

### [Patent Literature]

(Patent Literature 1) International Patent Publication No. 2009-046412
(Patent Literature 2) Korean Patent Registration No. 10-1639487

### [DETAILED DESCRIPTION OF THE INVENTION]

### [Technical Problem]

The present disclosure relates to a method for producing 1,4-cyclohexanedimethanol (CHDM) and, more specifically, provides a method for producing 1,4-cyclohexanedimethanol having a high proportion of trans isomers even without including an isomerization step, by adjusting the content of 1,4-cyclohexane dicarboxylic acid (CHDA) which is a reactant.

### [Technical Solution]

According to an embodiment of the present disclosure, there is provided a method for producing 1,4-cyclohexanedimethanol, the method comprising the step of subjecting a mixed solution containing 1,4-cyclohexane dicarboxylic acid (CHDA), a hydrogenation catalyst and water to a hydrogenation reaction to produce 1,4-cyclohexanedimethanol (CHDM) from the 1,4-cyclohexane dicarboxylic acid, wherein a content of the 1,4-cyclohexane dicarboxylic acid is 5 to 40 wt% based on the total amount of 1,4-cyclohexane dicarboxylic acid and water, and wherein the hydrogenation reaction is carried out at a temperature of 230 to 300°C.

According to another embodiment of the present disclosure, there is provided a composition comprising 1,4-cyclohexanedimethanol produced by the method for producing 1,4-cyclohexanedimethanol.

### [Advantageous Effects]

According to the preparation method of 1,4-cyclohexanedimethanol of the present disclosure, even if the hydrogenation reaction is carried out using 1,4-cyclohexane dicarboxylic acid as a starting material without going through an isomerization reaction step, 1,4-cyclohexanedimethanol having a high proportion of trans isomer can be produced by adjusting the content of 1,4-cyclohexanedicarboxylic acid and/or the reaction temperature.

According to the production method, since an additional isomerization step is not carried out, the process can be simplified and economical, and the trans isomer content of the produced CHDM is high, which can expect improvement of physical properties when used as a polymer raw material.

### [DETAILED DESCRIPTION OF THE EMBODIMENTS]

The technical terms used herein is only to explain embodiments and is not intended to limit the scope of the present disclosure. The singular forms "a,""an" and "the"are intended to include plural forms, unless the context clearly indicates otherwise. It should be understood that the terms "comprise," "include", "have", etc. are used herein to specify the presence of stated features, integers, steps, components or combinations thereof, but do not preclude the presence or addition of one or more other features, integers, steps, components, or combinations thereof.

Although the present disclosure may have various forms and various modifications may be made thereto, specific examples will be exemplified and explained in detail below. However, it is not intended to limit the present disclosure to specific disclosure, and it should be understood that the present disclosure includes all the modifications, equivalents or replacements thereof without departing from the spirit and technical scope of the present disclosure.

Now, the method for producing 1,4-cyclohexanedimethanol according to specific embodiments of the present disclosure will be described in more detail.

According to an embodiment of the present disclosure, there is provided a method for producing 1,4-cyclohexanedimethanol, the method comprising the step of subjecting a mixed solution containing 1,4-cyclohexane dicarboxylic acid (CHDA), a hydrogenation catalyst and water to a hydrogenation reaction to produce 1,4-cyclohexanedimethanol (CHDM) from the 1,4-cyclohexane dicarboxylic acid, wherein a content of the 1,4-cyclohexane dicarboxylic acid is 5 to 40 wt% based on the total amount of 1,4-cyclohexane dicarboxylic acid and water, and wherein the hydrogenation reaction is carried out at a temperature of 230 to 300°C.

Conventionally, for the production of 1,4-cyclohexanedimethanol with a high trans content, it essentially involved an isomerization reaction step to convert a cis isomer to a trans isomer. As an example, 1,4-cyclohexane dicarboxylic acid was used as a raw material for an isomerization reaction to obtain 1,4-cyclohexane dicarboxylic acid with a high trans content, and then this was used again as a raw material for hydrogenation reaction to produce 1,4-cyclohexanedimethanol with a high trans content, or alternatively, 1,4-cyclohexanedicarboxylic acid as a raw material was first hydrogenated, and then 1,4-cyclohexanedimethanol obtained was subjected to an isomerization reaction to produce 1,4-cyclohexanedimethanol with an improved trans content. However, when 1,4-cyclohexanedimethanol is produced by a conventional method, due to the additional isomerization process, the process is complicated and inefficient, and additional production costs are required, which is commercially undesirable.

Therefore, the method for producing 1 ,4-cyclohexanedimethanol according to an embodiment of the present disclosure adjusts the content and reaction temperature of 1,4-cyclohexanedicarboxylic acid as a reaction raw material, thereby making it possible to produce 1,4-cyclohexanedimethanol containing a high content of the trans isomer, even without including an isomerization reaction step of converting a cis isomer to a trans isomer.

When the hydrogenation reaction of a compound having cis/trans isomers progresses, it is generally expected that the cis and trans ratios of the reactants and products before and after the reaction are maintained without significant change. Apart from the fact that it can predict an improvement in the reaction rate even if the content of the reactant and the reaction temperature are increased, it is common that no change in the isomer ratio of the product due to the hydrogenation reaction is expected.

However, according to an embodiment of the present disclosure, when the content and reaction temperature of 1,4-cyclohexane dicarboxylic acid are within a certain range, it was possible to confirm an unexpected effect that the trans isomer content of the product 1,4-cyclohexanedimethanol is increased as compared with the reaction product even without including an additional isomerization reaction step.

More specifically, the method for producing 1,4-cyclohexanedimethanol according to an embodiment of the present disclosure is carried out by including the step of dissolving 1,4-cyclohexanedicarboxylic acid in water in the presence of a hydrogenation catalyst to produce a mixed solution, followed by a hydrogenation reaction. Wherein, the cis/trans ratio of 1,4-cyclohexanedicarboxylic acid, which is the reaction raw material, is not limited, but in one example, the proportion of the trans isomer in 1,4-cyclohexane dicarboxylic acid may be 20 to 35%, or 20 to 25%.

The content of 1,4-cyclohexane dicarboxylic acid may be 5 to 40 wt%, preferably 5 to 30 wt%%, 7 to 30 wt%, 7 to 25 wt%, 7 to 23 wt%, or 10 to 23 wt%, based on the total amount of 1,4-cyclohexane dicarboxylic acid and water.

When the content of 1,4-cyclohexane dicarboxylic acid is less than 5 wt% based on the total amount of 1,4-cyclohexane dicarboxylic acid and water, there are problems that the contact between the reactant and the catalyst is reduced and the reaction rate is slowed down, and the proportion of trans isomers in the produced CHDM is reduced. When it is greater than 40 wt%, the solubility of 1,4-cyclohexanedicarboxylic acid is reduced and productivity is reduced, and thus, the amount of crystals and catalysts in the reactants is increased, which can make the process of feeding the slurry difficult.

Meanwhile, the hydrogenation reaction may be carried out in a liquid phase or a gas phase. According to an embodiment of the present disclosure, the hydrogenation reaction may be progressed while the 1,4-cyclohexanedicarboxylic acid is a liquid phase dissolved in a solvent such as water, and hydrogen is a gaseous state.

Meanwhile, in order to minimize side reactions and optimize the ratio between reactants to improve process productivity, the amount of hydrogen introduced into a reactor may be 3 moles or more, or 4 moles or more, or 7 moles or more, and 300 moles or less, or 100 moles or less, or 50 moles or less, or 30 moles or less, based on 1 mole of 1 ,4-cyclohexanedicarboxylic acid.

If the amount of hydrogen is too small, i.e., less than 3 moles based on 1 mole of 1,4-cyclohexanedicarboxylic acid, the reaction conversion rate may decrease, and thus, a conversion rate of 95% or more cannot be obtained. If the amount is too large beyond 300 moles, the residence time of liquid drops of liquid raw materials in the reactor may be shortened due to hydrogen, and thus, conversion rate may decrease, by-products may increase, or catalyst life may rapidly decrease. From this point of view, the amount of hydrogen is preferably within the above range.

According to one embodiment of the present disclosure, the hydrogenation catalyst may include one or more metals selected from the group consisting of palladium (Pd), rhodium (Rh), ruthenium (Ru) and platinum (Pt) as an active ingredient, and one or more metals selected from the group consisting of tin (Sn), iron (Fe), rhenium (Re) and gallium (Ga), respectively.

Preferably, the hydrogenation catalyst may include ruthenium (Ru) and tin (Sn) as an active ingredient.

According to one embodiment of the present disclosure, the amount of the active ingredient of the hydrogenation catalyst can be appropriately controlled according to the content of the reactant CHDA. Specifically, as the content of the hydrogenation catalyst relative to CHDA is higher, a reaction rate increase, and thus, in the method for producing CHDM according to an embodiment of the present disclosure, the hydrogenation catalyst may be added in such an amount that the weight ratio of the hydrogenation catalyst to CHDA may become 0.01:1 or more.

However, if the content of the hydrogenation catalyst relative to CHDA is beyond a certain level, reaction rate increasing effect may be insignificant compared to the amount used, and thus, reaction efficiency may decrease. Considering this point, the first hydrogenation catalyst may be more specifically added in such an amount that the weight ratio of the hydrogenation catalyst to CHDA satisfies 0.01:1 to 3:1.

Considering the effect of improving the reaction rate by controlling the weight ratio of the hydrogenation catalyst to CHDA, the hydrogenation catalyst may be more preferably added in such an amount that a weight ratio of the hydrogenation catalyst to CHDA becomes 0.01: 1 to 3: 1, or 0.1: 1 to 3: 1, or 0.1: 1 to 2: 1.

However, the above weight ratio does not limit the range of the present disclosure, and the rate of the catalyst may be appropriately adjusted according to detailed reaction conditions and the kind of a reactor.

Such a hydrogenation catalyst can be used while being supported in a carrier, wherein as the carrier, those known in the art can be used without limitation. Specifically, carriers such as carbon, zirconia (ZrO₂), titania (TiO₂), alumina (Al₂O₃), or silica (SiO₂) can be used.

According to an embodiment of the present disclosure, when ruthenium (Ru) and tin (Sn) are included as active ingredients of the hydrogenation catalyst, ruthenium (Ru) and tin (Sn) may be included in an amount of 1 to 20 parts by weight, or 1 to 10 parts by weight, or 3 to 8 parts by weight, respectively, based on 100 parts by weight of the entire carrier.

When carbon is used as the carrier, although not specifically limited, at least one selected from the group consisting of active carbon, carbon black, graphite, graphene, OMC (ordered mesoporous carbon) and carbon nanotube can be used.

Preferably, it may be carbon black having high mesopore rate in the total pores, and as a specific example, the active carbon may be SXULTRA, CGSP, PK1-3, SX1G, DRACO S51HF, CA-1, A-51, GAS 1240 PLUS, KBG, CASP and SX PLUS, and the like, and the carbon black may be BLACK PEARLS^{®}, ELFTEX^{®}, VULCAN^{®}, MOGUL^{®}, MONARCH^{®}, EMPEROR^{®}, and REGAL^{®}, and the like, but not limited thereto.

Wherein, according to the present disclosure, in the carbon carrier, the volume rate of mesopores having pore size of 2 to 50 nm in the total pores may be 50% or more. Preferably, in the carbon carrier, the volume rate of mesopores in the total pores may be 70% or more, and more preferably in the carbon carrier, the volume rate of mesopores in the total pores may be 75% or more.

Wherein, if the volume rate of mesopores is less than 50%, there may be problems in terms of microscopic material transfer speed of reactant and product in the carbon carrier, and if the average size of the pores is greater than 50 nm, physical strength of the carrier may be weak, and thus, the above ranges are preferable.

Further, according to the present disclosure, the carbon includes ordered mesoporous carbon (OMC) having specific surface area (BET) of 100 to 1,500 m²/g. Preferably, the carbon may include ordered mesoporous carbon (OMC) having specific surface area (BET) of 200 to 1,000 m²/g. Wherein, if the specific surface area of carbon is less than 100 m²/g, it may be difficult for active metals (Ru, Sn) to be highly dispersed, and if the specific surface area of the carbon is greater than 1,500 m²/g, there may be a problem that the rate of mesopores decreases, and thus, the above range is preferable.

And, according to circumstances, the carbon carrier of the catalyst according to the present disclosure may include micropores in an appropriate rate, besides mesopores, and preferably, it may include 0 to 25 vol % of micropores based on the total pores. Wherein, if the volume rate of the micropores is greater than 25%, there may be a problem in terms of microscopic material transfer speed of reactant and product in the carbon carrier, and thus, the above range is preferable.

Although the hydrogenation reaction conditions are not specifically limited in the present disclosure, for example, the reaction temperature may be 230°C or more and 300°C or less, or 280 °C or less, or 270 °C or less. If the reaction temperature is less than 230°C, the contact between the reactant and the catalyst is reduced, or the reaction rate is lowered out of the operable temperature range of the catalyst, or the content of the trans isomer in the produced CHDM is reduced. If it is greater than 300°C, by-products may rapidly increase rapidly, and the catalyst life may be influenced, and thus, the above range is preferable.

Additionally, the reaction pressure may be 50 bar or more, or 80 bar or more, and 180 bar or less. If the reaction pressure is less than 50 bar, reactions may not sufficiently occur, and thus, an excessive amount of a catalyst may be consumed, and a residence time may too lengthen, thus increasing by-products, and if it is greater than 200 bar, excessive energy may be required during process operation, and manufacture cost of facilities such as a reactor may significantly increase, and thus, the above range is preferable.

And, during the hydrogenation reaction, a stirring process may be also conduced, and through the control of speed during the stirring process, hydrogenation reaction efficiency may be increased. Specifically, the stirring process may be conducted at a speed of 500 to 2,000 rpm, and more specifically, it is preferable that the stirring may be conducted at 700 to 1,500 rpm or 700 to 1,000 rpm.

Meanwhile, the stirring process may be conducted using a common stirrer.

It may be more preferable in terms of process efficiency that the process may be conducted for 1 to 10 hours under conditions fulfilling all the above hydrogenation reaction conditions.

According to one embodiment according to the present disclosure, in a mixed solution containing CHDA, hydrogenation catalyst and water, when a mixed solution containing 5 to 40 wt%, preferably 5 to 30 wt%, 7 to 30 wt%, 7 to 25 wt%, 7 to 23 wt%, or 10 to 23 wt% based on the total amount of 1,4-cyclohexane dicarboxylic acid and water is hydrogenated to produce CHDM, the proportion of trans CHDM in the total CHDM produced may be 63 wt% or more, 65 wt% or more, 67 wt% or more, 69 wt% or more, or 70 wt% or more, and the proportion of trans CHDM has not upper limit, but as an example, it may be 99 wt% or less, 95 wt% or less, 90 wt% or less, or 85 wt% or less.

Therefore, the CHDM finally obtained by the production method of the present disclosure has an excellent trans isomer content, and thus can be usefully used as a raw material for producing higher quality products.

Accordingly, another embodiment of the present disclosure provides a composition comprising CHDM produced by the method for producing 1,4-cyclohexanedimethanol.

Preferably, based on the total 1,4-cyclohexanedimethanol in the composition of one embodiment, the content of the trans isomer is 63 wt% or more, 65 wt% or more, 67 wt% or more, 69 wt% or more, or 70 wt% or more, and the proportion of trans CHDM has no upper limit, but as an example, it may be 99 wt% or less, 95 wt% or less, 90 wt% or less, or 85 wt% or less.

In addition, the composition of one embodiment can be used as a raw material for medicines, synthetic resins, synthetic fibers, or dyes.

Hereinafter, it will be described in more detail to assist in the understanding of the present disclosure. However, the following examples are for illustrative purposes only, and are not intended to limit the scope of the present disclosure.

### Example 1

A batch reactor that can withstand 300°C and 150 bar was selected as the reactor. The batch reactor is a device in which nitrogen for purging and hydrogen for hydrogenation reaction are introduced and can be stirred for the reaction. In the batch reactor, 18.8 g of reactant CHDA (trans-CHDA ratio: 21 %), 5.23 g of catalyst (ruthenium-tin/carbon catalyst, including 5 parts by weight of ruthenium, 5.8 parts by weight of tin, based on 100 parts by weight of the carbon carrier), and 250 g of distilled water as a solvent were added, purged twice with nitrogen of 5 bar, purged twice with hydrogen of about 5 bar, and the temperature was raised to 230°C while stirring at 50 rpm in a hydrogen atmosphere (about 14 to 15 bar).

When the reaction temperature was reached, hydrogen was injected up to the reaction pressure of 100 bar, then the stirring speed was increased to 1000 rpm, and the reaction was carried out for 6 hours.

### Example 2

The reaction was carried out in the same manner as in Example 1, except that 25 g of CHDA (trans-CHDA ratio: 21 %), 7 g of catalyst (ruthenium-tin / carbon catalyst, including 5 parts by weight of ruthenium and 5.8 parts by weight of tin based on 100 parts by weight of carbon carrier), and 225 g of distilled water as a solvent were used.

### Example 3

The reaction was carried out in the same manner as in Example 1, except that 50 g of CHDA (trans-CHDA ratio: 21%), 14 g of catalyst (ruthenium-tin / carbon catalyst, including 5 parts by weight of ruthenium and 5.8 parts by weight of tin based on 100 parts by weight of carbon carrier) were used.

### Example 4

The reaction was carried out in the same manner as in Example 1, except that the reaction temperature was 250°C.

### Comparative Example 1

The reaction was carried out in the same manner as in Example 1, except that 4.05 g of CHDA (trans-CHDA ratio: 21%), 1.125 g of catalyst (ruthenium-tin / carbon catalyst, including 5 parts by weight of ruthenium and 5.8 parts by weight of tin based on 100 parts by weight of carbon carrier), and 250 g of distilled water as a solvent were used.

### Comparative Example 2

The reaction was carried out in the same manner as in Example 1, except that the reaction temperature was 190°C.

### Comparative Example 3

The reaction was carried out in the same manner as in Example 1, except that the reaction temperature was 210°C.

### Experimental Example

Trans CHDM content was analyzed using Gas Chromatography (GC, column:
HP-5, detector: FID).

**[Table 1]**

| | Reaction temperature (°C) | Content of CHDA based on the total amount of CHDA and water | Ratio of trans CHDM in the product (%) |
|---|---|---|---|
| Com parative Example 1 | 230 | 1.6 | 56 |
| Com parative Example 2 | 190 | 7 | 50 |
| Com parative Example 3 | 210 | 7 | 56 |
| Example 1 | 230 | 7 | 67 |
| Example 2 | 230 | 10 | 71 |
| Example 3 | 230 | 20 | 74 |
| Example 4 | 250 | 7 | 75 |

Comparing Comparative Example 1 and Examples 1 to 3 in Table 1, it can be seen that the content of the trans isomer in the produced CHDM increases according to the concentration of CHDA under the same reaction conditions. In particular, it can be seen that when the concentration of CHDA is 7 wt% or more based on the total amount of 1,4-cyclohexanedicarboxylic acid and water, the proportion of trans CHDM is excellent as 67% or more.

In addition, comparing Comparative Examples 2 and 3 with Examples 1 and 4, it can be confirmed that when all other reaction conditions are the same, the proportion of CHDM in the product increases as the reaction temperature rises. In particular, it was found that when the reaction temperature was 230 °C or more, the ratio of trans CHDM in the product was excellent as 67% or more.

## Claims

1. A method for producing 1,4-cyclohexanedimethanol, the method comprising the step of subjecting a mixed solution containing 1,4-cyclohexane dicarboxylic acid (CHDA), a hydrogenation catalyst and water to a hydrogenation reaction to produce 1,4-cyclohexanedimethanol (CHDM) from the 1,4-cyclohexane dicarboxylic acid,
wherein a content of the 1,4-cyclohexane dicarboxylic acid is 5 to 40 wt% based on the total amount of 1,4-cyclohexane dicarboxylic acid and water, and
wherein the hydrogenation reaction is carried out at a temperature of 230 to 300 °C.

2. The method for producing 1,4-cyclohexanedimethanol according to claim 1, wherein
the method for producing 1,4-cyclohexanedimethanol does not include an isomerization reaction step of converting a cis isomer to a trans isomer.

3. The method for producing 1,4-cyclohexanedimethanol according to claim 1, wherein
the proportion of the trans isomer of 1,4-cyclohexanedicarboxylic acid relative to the total 1,4-cyclohexanedicarboxylic acid is 20 to 35%.

4. The method for producing 1,4-cyclohexanedimethanol according to claim 1, wherein
the content of the 1,4-cyclohexane dicarboxylic acid is 7 to 30 wt% based on the total amount of 1,4-cyclohexane dicarboxylic acid and water.

5. The method for producing 1,4-cyclohexanedimethanol according to claim 1, wherein
the hydrogenation catalyst comprises one or more metals selected from the group consisting of palladium (Pd), rhodium (Rh), ruthenium (Ru) and platinum (Pt), and one or more metals selected from the group consisting of tin (Sn), iron (Fe), rhenium (Re), and gallium (Ga), respectively.

6. The method for producing 1,4-cyclohexanedimethanol according to claim 1, wherein
the hydrogenation catalyst comprises ruthenium (Ru) and tin (Sn).

7. The method for producing 1,4-cyclohexanedimethanol according to claim 1, wherein
a weight ratio of the hydrogenation catalyst to 1,4-cyclohexane dicarboxylic acid is 0.01:1 to 3:1.

8. The method for producing 1,4-cyclohexanedimethanol according to claim 1, wherein
the hydrogenation reaction is carried out at a temperature of 230 to 270°C.

9. The method for producing 1,4-cyclohexanedimethanol according to claim 1, wherein
the hydrogenation reaction is carried out at 50 to 220 bar.

10. The method for producing 1,4-cyclohexanedimethanol according to claim 1, wherein
the content of the trans isomer in 1,4-cyclohexanedimethanol is 63 wt% or more.

11. A composition comprising 1,4-cyclohexanedimethanol produced by the method according to claim 1.

12. The composition according to claim 11, wherein
a content of the trans isomer in the total 1,4-cyclohexanedimethanol in the composition is 63 wt% or more.

13. The composition according to claim 11, wherein
the composition is a raw material for medicines, synthetic resins, synthetic fibers, or dyes.
